## Europäisches Patentamt

## European Patent Office

## Office européen des brevets

(19)

(11) Veröffentlichungsnummer : **0 049 801**
**B1**

(12)

# EUROPÄISCHE PATENTSCHRIFT

(45) Veröffentlichungstag der Patentschrift :
07.11.84

(21) Anmeldenummer : 81107714.8

(22) Anmeldetag : 29.09.81

(51) Int. Cl.³ : **C 12 N 9/90**, C 12 P 19/24,
C 12 P 19/12, C 12 N 11/00

(54) Saccharose-Mutase, immobilisierte Saccharose-Mutase und Verwendung von dieser immobilisierten Saccharose-Mutase zur Herstellung von Isomaltulose (6-0-alpha-D-Glucopyranosido-D-fructose).

(30) Priorität : 09.10.80 DE 3038218

(43) Veröffentlichungstag der Anmeldung :
21.04.82 Patentblatt 82/16

(45) Bekanntmachung des Hinweises auf die Patenterteilung : 07.11.84 Patentblatt 84/45

(84) Benannte Vertragsstaaten :
AT BE CH DE FR GB IT LI NL SE

(56) Entgegenhaltungen :
EP-A- 0 028 900
DE-A- 2 919 622
DE-B- 1 049 800
FR-A- 2 165 832
FR-A- 2 346 366
FR-A- 2 424 282
CHEMICAL ABSTRACTS, Band 90, Nr. 18, 30. April 1979, Zusammenfassung Nr. 142136f, Seite 273 COLUMBUS OHIO (US)
J. Amer. Chem. Soc. 77 (1955), 2412-2418
J. Amer. Chem. Soc. 78 (1956), 2514
J. Org. Chem. 25, (1960), 1060-1063
J. Biol. Chem. 200 (1953), 793-801

(73) Patentinhaber : BAYER AG
Konzernverwaltung RP Patentabteilung
D-5090 Leverkusen 1 Bayerwerk (DE)

(72) Erfinder : Kutzbach, Carl, Dr.
In den Birken 75 a
D-5600 Wuppertal 1 (DE)
Erfinder : Schmidt-Kastner, Günter, Dr. Prof.
Falkenberg 59
D-5600 Wuppertal 1 (DE)
Erfinder : Schutt, Hermann, Dr.
Gellertweg 12
D-5600 Wuppertal 1 (DE)

Jouve, 18, rue St-Denis, 75001 Paris, France

**Beschreibung**

Gegenstand der Erfindung ist das neue Enzym Saccharose-Mutase, das Saccharose in hoher Ausbeute in Isomaltulose umwandelt, wobei als wichtigste Nebenprodukte 1-O-α-D-Glucopyranosido-D-fructose, Fructose und Glucose jedoch keine nachweisbaren Mengen anderer Disaccharide entstehen, immobilisierte Saccharose-Mutase und die kontinuierliche Umwandlung von Saccharose in Isomaltulose (6-O-α-D-Glucopyranosido-D-fructose) mit Hilfe dieser immobilisierten Saccharose-Mutase.

Aus Isomaltulose kann durch Hydrierung nach DE-A 2 520 173 bzw. DE-C 2 217 628 ein Gemisch aus 6-O-α-D-Glucopyranosido-D-sorbit und 6-O-α-D-Glucopyranosido-D-mannit gewonnen werden, das als Zuckeraustauschstoff verwendet werden kann.

Es ist bekannt, daß Saccharose durch Zellen von Protaminobacter rubrum in guter Ausbeute in Isomaltulose umgewandelt werden kann (DE-A 1 049 800).

Nach DE-C 2 217 628 verläuft diese Umsetzung optimal in 15-40 %iger Saccharose-Lösung bei 20-37 °C und starkem Rühren und Belüften.

Nach DE-A 2 806 216 kann die Vermehrung der Bakterien und die Umwandlung von Saccharose in Isomaltulose gleichzeitig und kontinuierlich durchgeführt werden.

Statt Protaminobacter rubrum können in solchen Verfahren auch andere Bakterien, wie Erwinia carotovora, Serratia marcescens (NCIB 8285), Serratia plymuthica (ATCC 15 928) und Leuconostoc mesenteroides (NRRL B-512f) (ATCC 10830a) verwendet werden.

Diese Fermentationsverfahren haben verschiedene Nachteile :

1. Für die Vermehrung der Zellen wird ein Teil der eingesetzten Saccharose als Kohlenstoff-Quelle verbraucht.

2. Die für die Zellvermehrung notwendige Rührung und Belüftung erfordert einen hohen Energieaufwand.

3. Bei der Abtrennung der Zellen geht ein Teil des Produktes verloren.

4. Die optimale Saccharosekonzentration liegt nach DE-OS 2 806 216 bei ca. 25 %. Der Zucker muß demnach zuerst auf diese Konzentration verdünnt und nach der Umsetzung zur Kristallisation des Produktes mit hohem Energieaufwand wieder aufkonzentriert werden.

5. Die Vermehrung der Bakterien erfordert neben der Kohlenstoff-Quelle Saccharose stickstoffhaltige Substanzen und Salze, deren Anwesenheit bei der Kristallisation des Produktes zu Verlusten führt.

Alle diese Nachteile sind prinzipiell vermeidbar, wenn es gelingt, die Umwandlung der Saccharose in Isomaltulose von der Zellvermehrung zu trennen und über längere Zeit zu stabilisieren.

Aus EP-A-28 900, insbesondere in Beispiel 9, ist von der Gewinnung und Reinigung eines Enzyms aus Erwinia rhapontici die Rede. Eine genauere Betrachtung dieses Beispiels zeigt jedoch, daß dieses Ziel durchaus nicht erreicht wurde.

Aus J. Amer. Chem. Soc. *77* (1955), 2412-2418, J. Amer. Chem. Soc. *78* (1956), 2514, J. Org. Chem. *25* (1960), 1060-1063 und J. Biol. Chem. *200* (1953), 793-801, ist ein Enzym Dextransucrase aus Leuconostoc mesenteroides bekannt. Da es jedoch ohne Zellaufschluß gewonnen wird, muß angenommen werden, daß es sich im Gegensatz zu der erfindungsgemäßen Saccharose-Mutase um ein Exoenzym handelt.

Es wurde nun überraschenderweise gefunden, daß man aus den Zellen von Isomaltulose-bildenden Bakterien, insbesondere Protaminobacter rubrum CBS 57 477, ein Enzym gewinnen kann, das hochkonzentrierte Lösungen von reiner Saccharose in sehr guter Ausbeute in Isomaltulose umwandelt.

Bei dieser Umwandlung entstehen als wichtigste Nebenprodukte 1-O-α-D-Glucopyranosido-D-fructose, Fructose und Glucose, jedoch keine nachweisbare Mengen anderer Disaccharide.

Das isolierte Enzym kann nach verschiedenen, grundsätzlich bekannten Verfahren immobilisiert und stabilisiert werden, womit eine kontinuierliche Umwandlung von Saccharoselösungen in einer Konzentration von 10 bis zu 75 Gew.-% bei 30 bis 60 °C durchgeführt werden kann.

Die Isolierung des Enzyms war insbesondere deshalb überraschend, weil Enzyme mit ähnlicher Aktivität, d. h. Mutasen, die eine intramolekulare Transfer-Reaktion an Oligosacchariden bewirken, bisher nicht bekannt geworden sind. Soweit bekannt, werden Disaccharide in der lebenden Zelle entweder durch Abbau von höheren Polymeren oder durch energieabhängige Synthese über Nucleotid-Derivate gebildet. Das isolierte Enzym ist als Saccharose-Mutase (Saccharose → Isomaltulose-Mutase) zu bezeichnen.

Das erfindungsgemäße Verfahren gliedert sich in folgende vier Schritte :

1. Vermehrung der Zellen ;
2. Isolierung des Enzyms ;
3. Immobilisierung des Enzyms ;
4. Umwandlung von Saccharose in Isomaltulose mit dem immobilisierten Enzym.

Zur Isolierung des Enzyms wurden insbesondere Zellen des Protaminobacter rubrum (CBS 57 477) in grundsätzlich bekannter Weise gezüchtet : Für den Zweck optimaler Enzymproduktion verwendet man vorteilhaft ein Medium aus Saccharose-haltigem Dicksaft, der auf einen Gehalt von 5-25 % Trockensubstanz verdünnt wird mit Zusatz von 2 % Maisquellwasser und 0,1 % $(NH_4)_2HPO_4$.

Man erreicht damit in einer Fermentationszeit von 14-16 Stunden bei 30 °C eine Ausbeute von 10-50 g sedimentierte Zellen/Liter Fermentationslösung und eine enzymatische Aktivität von 10-30 E/ml (1 E (Einheit) ≙ Umwandlung von 1 µMol Saccharose/min bei 30 °C ; zum Test siehe Beispiel 1b).

Die Isolierung des Enzyms erfolgt nach Konzentrierung und Aufschluß der Zellen nach allgemein bekannten Methoden der Enzymreinigung. Folgende Vorgehensweise hat sich besonders bewährt :

Die Zellen werden in einer Röhrenzentrifuge oder einem selbstentschlammenden Separator auf das ca. 20-fache konzentriert. Zur Freisetzung des Enzyms aus dem Zellinneren werden die konzentrierten Zellen, z. B. durch einen Hochdruckhomogenisator (Manton-Gaulin) bei 500 bar aufgeschlossen.

Die so erhaltene Lösung des Enzyms mit suspendierten Zelltrümmern kann, gegebenenfalls nach Einstellung eines vorteilhaften pH-Wertes zur Ausfällung von Verunreinigungen (z. B. pH 5), durch Zentrifugation oder Filtration geklärt werden. Diese geklärte Lösung des Enzyms kann bereits zur Immobilisierung verwendet werden. Für die meisten Immobilisierungs-Verfahren ist es jedoch vorteilhafter, das Enzym zuvor noch weiter anzureichern. Dazu eignen sich Fällungsverfahren, z. B. mit Ammoniumsulfat oder Polyethylglykol, und chromatographische Verfahren, z. B. Molekularsiebchromatographie (Gelchromatographie), Ionenaustauscher-Chromatographie und Adsorptionschromatographie. Als besonders wirkungsvoll hat sich die Chromatographie an CM-Sephadex ® (Carboxymethylsubstituiertes quervernetztes Dextran) erwiesen. Eine praktische Ausführung ist in Beispiel 1c beschrieben. Durch einmalige Chromatographie wird eine spezifische Aktivität des Enzyms von 50-250 E/mg Protein erreicht. Durch Wiederholung der Chromatographie an CM-Sephadex wird elektrophoretisch nahezu einheitliches Enzym mit einer spezifischen Aktivität von 450 E/mg gewonnen.

Das gereinigte Enzym wandelt in hoher Ausbeute Saccharose in Isomaltulose an. Zum Beispiel wurden aus einer 48 %igen Saccharose-Lösung bei 35 °C 86,5 % Isomaltulose, 8,6 % 1-O-α-D-Glucopyranosido-D-fructose, 4,0 % Fructose und 0,9 % Glucose erhalten. Andere charakteristische Eigenschaften des neuen Enzyms sind :

| Molmasse : | ca. 50 000 (durch Gelchromatographie) |
|---|---|
| Isoelektrischer Punkt : | 8,0 ± 0,3 |
| pH-Optimum : | pH 6,5 ± 0,5 |
| Stabilitäts-Optimum : | pH 5,5 ± 0,5. |

Das freie Enzym wird bei Temperaturen > 40 °C rasch inaktiviert. Die Reaktion wird durch CU- + Zn-Ionen stark gehemmt. Ca, $Mn^{2+}$, Mg und Borat haben keinen Einfluß auf die enzymatische Aktivität.

Die Immobilisierung des Enzyms kann nach einer Vielzahl von grundsätzlich bekannten Verfahren durchgeführt werden, wie sie z. B. in einer Zusammenfassung von I. Chibata : Immobilized. Enzymes, Halsted Press (A Division of I. Wiley & Sons) New York 1978, beschrieben sind.

In systematischer Weise kann man folgende Immobilisierungsverfahren unterscheiden, die alle grundsätzlich für die Immobilisierung der Saccharose-Mutase angewendet werden können :

Einschluß-Verfahren ;
Vernetzungs-Verfahren ;
Bindung an feste Träger durch
  Adsorption ;
  Kovalente Bindung ;
  Vernetzung ;
Bindung an Membranen :
Bindung an lösliche Träger.

Ein Beispiel für Einschluß-Verfahren ist der Einschluß der Enzymlösung in semipermeable Hohlfasern mit einer Porengröße, die den Zuckermolekülen den Durchtritt gestattet, die Enzymmoleküle jedoch im Inneren der Hohlfasern zurückhält. Eine praktische Ausführung dieses Verfahrens ist in Beispiel 2 beschrieben.

Als feste Träger für die Adsorption des Enzyms eignen sich z. B. Ionenaustauscher. Als besonders vorteilhaft haben sich Kationenaustauscher auf Polysaccharid-Basis gezeigt, z. B. CM-Cellulose (Beispiele 3 und 4). Die adsorptive Bindung ist so fest, daß selbst hohe Saccharose-Konzentrationen, z. B. 60 %, das Enzym nicht vom Träger ablösen. Es ist auch möglich, die Bindung durch Quervernetzung, z. B. mit Glutardialdehyd noch fester zu machen (Beispiel 5).

Eine besonders feste Bindung wird durch kovalente Verknüpfung erreicht. An Träger mit Hydroxylgruppen oder Aminogruppen kann eine kovalente Bindung, z. B. mit Reagentien wie Bromcyan, Cyanurchlorid oder FCP (Trifluorchlor-Pyrimidin) durchgeführt werden. Beispiele sind Bindungen an Cellulose und Sepharose mit verschiedenen Reagentien (Beispiele 6-8), an Polymere mit Anhydridgruppen (Beispiel 9) und an Silikat (Beispiel 10).

Aus kinetischen Gründen ist die Verwendung von membrangebundenen Enzymen besonders vorteilhaft (DE-A 2 553 649). Praktische Ausführungsformen der Bindung von Saccharose-Mutase an Membranen sind in den Beispielen 11 und 12 beschreiben.

Vorteilhaft ist auch die Immobilisierung durch kovalente Bindung an lösliche Träger, z. B. lösliche Stärke. Das Enzym wird dadurch stabilisiert und kann infolge des hohen Molgewichts des Trägers auf einfache Weise mit semipermeablen Membranen von der Isomaltulose-Lösung getrennt und wieder verwendet werden (Beispiel 13-17).

Die zweckmäßige Durchführung der Umwandlung von Saccharose in Isomaltulose mit dem nach einer der vorgenannten Methoden immobilisierten Enzym Saccharose-Mutase richtet sich nach den speziellen Charakteristika des immobilisierten Enzyms. In allen Fällen wird eine 40-70 %ige Lösung von Saccharose bei 30-60 °C mit dem immobilisierten Enzym solange in Kontakt gebracht, bis der gewünschte Grad der Umsetzung erreicht ist. Typischerweise erhält man, wenn die Kontaktzeit für vollständigen Verbrauch der Saccharose gerade ausreichend ist, ein Produktgemisch der Zusammensetzung 5 % Fructose, 2 % Glucose, 9 % 1-O-α-D-Glucopyranosido-D-fructose und 84 % Isomaltulose. Eine wesentlich verlängerte Kontaktzeit führt zu einer geringeren Ausbeute an Isomaltulose und höheren Ausbeuten an Fructose, Glucose und 1-O-α-D-glucopyranosido-D-fructose. Die Bildung dieser Nebenprodukte wird auch durch hochgereinigte Präparate der Saccharose-Mutase in nahezu unverändertem Mengenverhältnis katalysiert und ist deshalb als eine charakteristische Eigenschaft dieses Enzyms anzusehen. Die Saccharose-Konzentration und die Reaktionstemperatur haben nur geringen Einfluß auf das Produktspektrum.

Verwendet man für die Umwandlung der Saccharose in Isomaltulose das freie oder an lösliche Träger gebundene Enzym, so kann man die Reaktion z. B. in einem Hohlfaser-Reaktor (Beispiel 2) durchführen.

Eine andere Möglichkeit ist ein durch eine semipermeable Membran abgeschlossenes Gefäß, in dem man die Reaktion entweder kontinuierlich oder chargenweise durchführen kann.

An feste Träger gebundenes Enzym kann ebenfalls für chargenweisen Betrieb, z. B. in Rührkesseln oder für kontinuierlichen Betrieb in Säulen-Reaktoren mit aufsteigendem oder absteigendem Durchfluß, eingesetzt werden. Derartige Säulen-Reaktoren müssen temperierbar sein und haben typischerweise ein Durchmesser : Füllhöhen-Verhältnis von 1 : 1 bis 1 : 10.

An Membranen gebundenes Enzym kann als Flachmembran, Hohlfasermembran oder Filterpatrone vorliegen. Die mit Enzym beladene Membran wird in einer geeigneten temperierbaren Halterung von der Saccharose-Lösung einmal oder mehrmals durchströmt, bis der gewünschte Umsatz erreicht ist.

Aus den auf eine dieser Arten umgesetzten Saccharoselösungen kann ein Teil der gebildeten Isomaltulose durch Abkühlen kristallin gewonnen werden. Durch Eindampfen der Mutterlauge kann man eine weitere Menge Isomaltulose zur Kristallisation bringen, insgesamt können so 90-92 % der gebildeten Isomaltulose gewonnen werden.

## Beispiele

### Beispiel 1

Herstellung des Enzyms

1a) Fermentation von Protaminobacter rubrum :

Eine Abimpfung des Stammes Protaminobacter rubrum (CBS 57 477) wird in 200 ml einer Nährlösung aus auf 25 % Trockensubstanz verdünntem Dicksaft mit Zusatz von 0,5 g/l $(NH_4)_2HPO_4$, eingestellt auf pH 7,2 in einem 1-Liter-Kolben 16 Stunden bei 30 °C auf der Schüttelmaschine bei 290 U/min angezogen. Die Kultur aus 5-10 solcher Kolben (1-2 Liter) wird zur Beimpfung eines 300-Liter-Fermenters eingesetzt. Das Medium besteht aus 200 Litern mit Wasser auf 25 % Trockensubstanz verdünntem Dicksaft (ca. 94 % Saccharose in der Trockensubstanz) mit Zusatz von 0,5 g/l $(NH_4)_2HPO_4$ und 2 % Maisquellwasser.

Die Fermentation wird bei 30 °C ohne pH-Kontrolle mit einer Belüftung von 100 l/min und einer Rührgeschwindigkeit von 180 U/min durchgeführt und ist nach 16-18 Stunden beendet.

1b) Bestimmung der Enzymaktivität

Zur Bestimmung der Enzymaktivität wird ein Inkubationsansatz aus 5 ml einer 10 % Saccharose Lösung in 0,01 M Phosphatpuffer pH 7,0 verwendet. Nach Zugabe von 1-10 E des zu testenden Enzympräparates (Zellsuspension, Enzymlösung oder immobilisiertes Enzym) wird 60 Minuten bei 30 °C unter Schütteln inkubiert. Dann beendet man die Reaktion durch Erhitzen auf 100 °C für eine Minute. Eine Kontrollprobe wird direkt nach Zugabe des Enzympräparates durch Erhitzen auf 100 °C abgestoppt.

Die Bestimmung des Umsatzes kann nach 3 Methoden durchgeführt werden :

1. Bestimmung der nicht umgesetzten Saccharose und der Produkte durch HPLC (Hochdruckflüssigkeitschromatographie).

2. Enzymatische Bestimmung der nicht umgesetzten Saccharose, z. B. nach H. U. Bergmeyer und E. Bernt in H. U. Bergmeyer, Methoden der enzymatischen Analyse, 2. Auflage, Band II, Seite 1143-1146, Verlag Chemie 1970.

3. Durch Bestimmung der gebildeten reduzierenden Zucker mit Dinitrosalizylsäure, da Saccharose nicht reduziert, während die Produkte der Reaktion reduzierend wirken. Das Reagenz besteht aus einer wäßrigen Lösung von 10 g Dinitrosalizylsäure, 16 g Natriumhydroxid und 300 g K, Na-tartrat in 1 Liter. Zur Durchführung der Reaktion werden 0,4 ml des Inkubationsansatzes mit 0,5 ml dieses Reagenz versetzt, 5 Minuten auf 100 °C erhitzt, mit Wasser abgekühlt, mit 2,5 ml Wasser verdünnt und die Extinktion bei 540 nm in einem Fotometer gegen einen Reagentien-0-Wert bestimmt. Die Auswertung erfolgt mittels einer Standardkurve, die mit reiner Isomaltulose gemessen wurde. Man erhält die gebildeten reduzierenden Zucker als Isomaltulose-Äquivalente in μMol/ml des Inkubationsansatzes und errechnet daraus die enzymatische Aktivität nach der Formel :

$$E/ml \text{ oder } g = (\mu Mol/ml \times 5)/(60 \times \text{Probenmenge Enzym (ml oder g))}$$

### 1c) Isolierung des Enzyms

Aus 200 Litern Fermentationslösung nach Beispiel 1a mit einer Aktivität von 11,5 E/ml werden durch Zentrifugation in einer Röhrenzentrifuge (Sharples) 28 Liter Bakterienschlamm mit einer Aktivität von 77,5 E/ml (94 % Ausbeute) erhalten. Der Bakterienschlamm wird mit entsalztem Wasser auf 50 Liter verdünnt und der pH-Wert durch Zugabe von verdünnter Natronlauge auf pH 5,5-6,0 gehalten. Der verdünnte Schlamm wird in einem Hochdruck-Homogenisator (Manton-Gaulin) bei 500 bar mit einem Durchfluß von 20 Litern/Stunde aufgeschlossen.

Auschließend wird mit Essigsäure ein pH-Wert von 5,0 eingestellt und durch Zentrifugation in der Röhrenzentrifuge die Enzymlösung von Zelltrümmern und präzipitierten Verunreinigungen befreit. Das noch trübe Zentrifugat wird durch Filtration, z. B. über ein Seitz-AS-Filter weiter geklärt. Man erhält 44 Liter Enzymlösung mit 43 E/ml (87 % Ausbeute). Die Lösung enthält 19,1 mg Protein/ml (Lowry-Methode). Die spezifische Aktivität des Enzyms beträgt 2,15 E/mg.

756 ml (32 500 E) der geklärten Lösung werden mit verdünnter Natronlauge auf pH 6,0 eingestellt und zur Einstellung einer Leitfähigkeit von 4,5 mS mit entsalztem Wasser auf 3 250 ml verdünnt. Diese Lösung wird auf eine Säule 11 × 30 cm (2,9 Liter) mit CM-Sephadex C-50, äquilibriert mit Kaliumphosphatpuffer pH 6,0 4,5 mS aufgetragen. Dann wäscht man zur Entfernung von Verunreinigungen mit 3 Litern des gleichen Puffers nach. Anschließend wird mit einem linearen Gradienten aus 5 Litern des Waschpuffers und 5 Litern des gleichen Puffers mit Zusatz von 0,5 M NaCl eluiert. Es werden Fraktionen von 25 ml aufgefangen. Die Saccharose-Mutase-Aktivität erscheint in den Fraktionen 150-200. Die vereinigten aktiven Fraktionen enthalten in 1230 ml 27 122 E (83,5 %) mit einer spezifischen Aktivität von 98 E/mg. Die Enzymlösung wird durch Ultrafiltration über eine Membran mit Ausschlußgrenze 20 000 Dalton (Amicon UM-20 E) auf 475 ml konzentriert. Das Konzentrat wird über eine Säule von Polyacrylamid-Gel (Biorad Biogel P4) mit dem Elutionsmittel Wasser entsalzt. Ausbeute 21 535 E (79 %) mit 107 E/mg.

### Beispiel 2

### Einschluß des Enzyms in Hohlfasern

Ein Bündel aus semipermeablen Hohlfasern (Typ H1P10 Fa. Amicon) wird mit 23 ml einer Lösung von Saccharose-Mutase, enthaltend 1905 E mit einer spezifischen Aktivität von 48,7 E/mg, gefüllt. Durch das Innere der Hohlfasern leitet man eine 10 %ige Saccharose-Lösung. Bei 25 °C findet man in der ablaufenden Lösung durch HPLC-Analyse folgendes Produktgemisch :

Bei 60 ml/Stunde :
4 % Fructose, 3 % Glucose, 1 % Saccharose, 86 % Isomaltulose und 6 % 1-O-α-D-glucopyranosido-D-fructose.

Bei 100 ml/Stunde :
4 % Fructose, 4 % Glucose, 7 % Saccharose, 76 % Isomaltulose, 9 % 1-O-α-D-glucopyranosido-D-fructose.

Diese Ergebnisse waren auch nach 5-tägigem Dauerbetrieb praktisch unverändert.

### Beispiel 3

### Adsorptive Bindung an CM-Cellulose

121 ml einer Lösung von Saccharose-Mutase, enthaltend 5810 E mit einer spezifischen Aktivität von 49 E/mg werden mit 50 g feuchter auf pH 6,0 äquilibrierter CM-Cellulose (Typ CM 52, Fa. Whatman) versetzt. Die Leitfähigkeit der Enzymlösung liegt bei 4,2 mS. Die Suspension wird 2 Stunden bei Raumtemperatur gerührt und das an den Träger adsorbierte Enzym abgenutscht.
Ausbeute : 52,0 E/g ≙ 2965 E (51 %).
Im Filtrat finden sich 759 E (13 %).

## Beispiel 4

Adsorptive Bindung an CM-Cellulose

In gleicher Weise werden 222 ml einer Lösung von Saccharose-Mutase, enthaltend 4770 E (77 E/mg), mit 25 g CM-Cellulose (Typ CM 11, Fa. Whatman) bei pH 5,5 umgesetzt.
Ausbeute : 26,6 g mit 50,9 E/g $\triangleq$ 1354 E $\triangleq$ 28 %.
Im Filtrat werden 730 E (15 %) gefunden.

## Beispiel 5

Adsorption mit anschließender Vernetzung

10 g des trägeradsorbierten Enzyms aus Beispiel 3 werden 1 Stunde mit einer Lösung von 50 ml 0,2 % Glutardialdehyd verrührt und dann abgenutscht. Ausbeute : 10,4 g mit einer Aktivität von 49,2 E/g (98 %).

## Beispiel 6

Bromcyankupplung von Saccharose-Mutase an Sepharose 4B CL

3 g feuchte Sepharose 4B CL werden mit 50 mg Bromcyan 30 Minuten bei pH 11,0 aktiviert, der pH-Wert der Suspension mit 1-n HCl auf pH 7,0 eingestellt und 2 Stunden bei pH 7,0 mit 25 ml Saccharose-Mutase-Lösung (enthaltend 6350,7 Einheiten und 30,62 mg Protein nach Lowry) gerührt. Der Enzymträger wird filtriert und mit destilliertem Wasser gewaschen.
Ausbeute an Enzymträger : 2,7 g mit 50,0 Einheiten/g = 135 Einheiten $\triangleq$ 2,1 % der eingesetzten Aktivität.
Filtrat : 120 ml mit 6 486 Einheiten.

## Beispiel 7

Kovalente Bindung von Saccharose-Mutase an Sepharose 4B CL® nach Aktivierung mit Cyanurchlorid

90 g feuchte Sepharose 4B CL® werden 30 Minuten lang mit 1,5 g Cyanurchlorid in einem Gemisch aus 100 ml destilliertem Wasser und 100 ml Dioxan bei pH 6-7 aktiviert. Die aktivierte Sepharose 4B wird abgesaugt, mit Dioxan und destilliertem Wasser gewaschen und zuletzt in 100 ml destilliertem Wasser suspendiert. 59 ml Saccharose-Mutase-Lösung mit 6 661 Einheiten und 32,1 mg Protein nach Lowry werden zugefügt und 4 Stunden bei Raumtemperatur und pH 7,0 gerührt. Der Enzymträger wird abgesaugt und auf der Nutsche mit destilliertem Wasser gewaschen.
Ausbeute an Enzymträger : 93,2 g mit 18,54 Einheiten/g = 1 728 Einheiten $\triangleq$ 27,2 % der eingesetzten Aktivität.
Filtrat : 280 ml mit 85,7 Einheiten = 1,35 % der eingesetzten Aktivität.

## Beispiel 8

Kovalente Bindung von Saccharose-Mutase an Cellulose nach Aktivierung mit Cyanurchlorid

50 g Cellulose (Avicel der Fa. Merck, Darmstadt) werden wie in Beispiel 7 beschrieben mit 5 g Cyanurchlorid aktiviert. Es werden zum aktivierten Träger 25 ml Saccharose-Mutase-Lösung mit 6 350,7 Einheiten und 30,62 mg Protein nach Lowry zugefügt und wie unter 2.2. angegeben aufgearbeitet.
Ausbeute an Enzymträger : 119,7 g mit 15,5 Einheiten/g = 1 850,7 Einheiten $\triangleq$ 29,1 % der eingesetzten Aktivität.
Filtrat : 275 ml mit 39,7 Einheiten = 0,63 % der eingesetzten Aktivität.

## Beispiel 9

Kovalente Bindung von Saccharose-Mutase an Anhydridharz.

10 g Anhydridharz (Copolymerisat aus 80 Gew.-% Tetraethylenglykoldimethacrylat, 10 Gew.-% Maleinsäure und 10 Gew.-% Maleinsäureanhydrid, Korngröße : 0,5-0,8 mm) werden in Aceton suspendiert und noch mehrmals auf einer Fritte mit Aceton gewaschen. Das gewaschene Harz wird in 20 ml destilliertem Wasser suspendiert und über Nacht bei pH 6,2 mit 66 ml Saccharose-Mutase-Lösung (2 003,1 Einheiten und 19,8 mg Protein nach Lowry) gerührt. Der Enzymträger wird abgesaugt und auf der Fritte mit destilliertem Wasser, 10 %iger NaCl-Lösung und wieder mit destilliertem Wasser gewaschen.
Ausbeute an Enzymträger : 14,48 g mit 18,9 Einheiten/g = 273,5 Einheiten $\triangleq$ 13,6 % der eingesetzten Aktivität.
Filtrat : 315 ml mit 85,1 Einheiten = 4,27 % der eingesetzten Aktivität.

6

# 0 049 801

Beispiel 10

Kovalente Bindung von Saccharose-Mutase an Silikat

500 ml Natronwasserglas (Fa. Merck, Darmstadt) werden unter Rühren mit ca. 100 ml 50 %iger Schwefelsäure versetzt, der Niederschlag abgesaugt, trockengepreßt und im Umlufttrockenschrank bei 160-180 °C getrocknet. Das erhaltene Silikat wird im Mörser zerkleinert.

50 g Silikat werden zur Einführung von Aminogruppen 8 Stunden mit 1,33 Litern einer 10 %igen Lösung von γ-Aminopropyltriethoxysilan in Benzol am Rückfluß gekocht und nach Abkühlung mit je 330 ml Benzol und 330 ml Aceton je dreimal gewaschen. Das Aminogruppen tragende Silikat wurde mit ca. 1 Liter 0,05 M Phosphatpuffer pH 7,0 portionsweise gewaschen.

Ausbeute : 39,0 g Amino-Silikat

19,5 g Amino-Silikat werden in 200 ml destilliertem Wasser/Dioxan (1 : 1) suspendiert und mit 1,95 g Cyanurchlorid 60 Minuten bei pH 5-7 aktiviert. Das aktivierte Harz wird mit Dioxan und destilliertem Wasser gewaschen. Unter Rühren werden 100 ml Saccharose-Mutase-Lösung mit 15 030 Einheiten zugefügt und die Suspension 4 Stunden bei Raumtemperatur gerührt. Der Enzymträger wird abgesaugt und auf der Nutsche gewaschen.

Ausbeute an Enzymträger : 25,1 g mit 58,7 Einheiten/g = 1 473 Einheiten ≙ 9,8 % der eingesetzten Aktivität.

Filtrat : Keine Aktivität.

Beispiel 11

Isomerisierung der Saccharose zur Isomaltulose in einem Enzym-Membran-Reaktor

a) Eine Filtercartridge mit einer Silikat-Polymer-Membran, z. B. eine Amerace H30-Cartridge mit einer Membran-Oberfläche von 0,25 m², wird in ein entsprechendes Filtergehäuse eingesetzt und mittels einer Pumpe mit destilliertem Wasser bei Zimmertemperatur vorgewaschen. In den Poren einer so vorbehandelten Membran-Cartridge wird das Enzym durch Umpumpen einer verdünnten Saccharose-Mutase-Lösung (25 E/ml) bei + 4 °C adsorptiv-immobilisiert. Der Rest der Enzym-Lösung wird mit destilliertem Wasser bei + 4 °C herausgewaschen und der Enzym-Membran-Reaktor feucht gelagert.

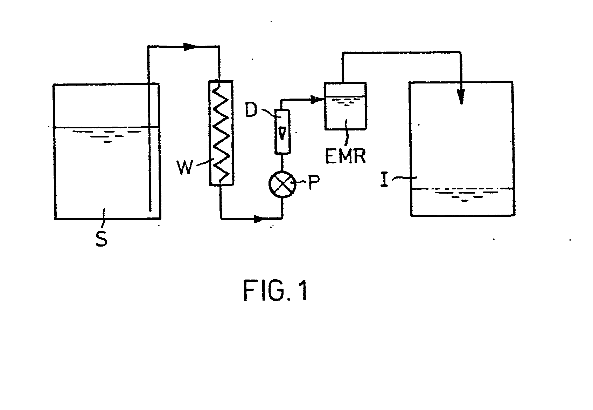

b) Zur Isomerisierung der Saccharose zur Isomaltulose wird nach Abb. 1 eine filtrierte 10 %ige Saccharose-Lösung aus dem Behälter (S) mittels einer stufenlos regelbaren Pumpe (P) über einen Wärmeaustauscher (W) durch einen Durchflußmesser (D) bei 34 °C kontinuierlich durch den Enzym-Membran-Reaktor (EMR) in den Isomaltulose-Behälter (I) gepumpt. Die Zusammensetzung der Isomaltulose-Lösung wird durch HPLC bestimmt.

Die Umsatzrate ist gemäß nachstehender Tabelle 1 abhängig von der Durchflußgeschwindigkeit.

Tabelle 1

Zusammensetzung der Isomaltulose-Lösung in Abhängigkeit von der Durchflußgeschwindigkeit (0,25 m² Membranoberfläche ; 10 %ige Saccharose-Lösung).

| Durchflußgeschwindigkeit in Ltr./h | Isomaltulose % | Rest-Saccharose % |
|---|---|---|
| 0,68 | 72,6 | 0,0 |
| 1,9 | 70,3 | 9,4 |
| 4,2 | 65,3 | 16,1 |
| 8,2 | 31,4 | 59,1 |

Beispiel 12

Isomerisierung der Saccharose zur Isomaltulose in einem Enzym-Membran-Reaktor

a) Eine Filtercartridge mit einer Silikat-Polymer-Membran, z. B. eine Amerace H30®-Cartridge mit einer Membran-Oberfläche von 0,25 m², wird in ein entsprechendes Filtergehäuse eingesetzt und mittels einer Pumpe mit destilliertem Wasser bei Zimmertemperatur vorgewaschen. In den Poren einer so vorbehandelten Membran-Cartridge wird das Enzym durch Umpumpen einer verdünnten Saccharose-Mutase-Lösung (25 E/ml) bei + 4 °C adsorptiv-immobilisiert. Der Rest der Enzym-Lösung wird mit destilliertem Wasser bei + 4 °C herausgewaschen. Der Enzym-Membran-Reaktor wird feucht gelagert.

7

b) Zur Isomerisierung der Saccharose zur Isomaltulose wird nach Abb. 1 eine filtrierte 20 %ige Saccharose-Lösung aus dem Behälter (S) mittels einer stufenlos regelbaren Pumpe (P) über einen Wärmeaustauscher (W) bei 34 °C durch einen Durchflüßmesser (D) kontinuierlich durch den Enzym-Membran-Reaktor (EMR) in den Isomaltulose-Behälter gepumpt. Der Gehalt der Isomaltulose-Lösung wird durch HPLC bestimmt. Nach Tabelle 2 ist die Umsatzrate abhängig von der Durchflußgeschwindigkeit. Bei einer Durchflußgeschwindigkeit von 0,58 Ltr. pro Stunde und bei einem Isomaltulose-Gehalt von 74,1 % (= 148,2 g/Ltr.) berechnet sich eine Kapazität des Enzymmembran-Reaktors von ca. 8,3 kg Isomaltulose pro m² Membranfläche pro Tag.

Tabelle 2

Zusammensetzung der Isomaltulose-Lösung in Abhängigkeit von der Durchflußgeschwindigkeit (0,25 m² Membranoberfläche ; 20 %ige Saccharose-Lösung).

| Durchflußgeschwindigkeit in Ltr./h | Isomaltulose % | Rest-Saccharose % |
|---|---|---|
| 0,58 | 74,1 | 0,0 |
| 1,1 | 72,8 | 2,8 |
| 2,3 | 69,1 | 14,7 |
| 3,9 | 41,2 | 48,7 |

Beispiel 13

Kovalente Bindung von Saccharose-Mutase an Stärke nach Aktivierung mit Bromcyan

2 g Stärke nach Zulkowsky (Fa. Merck, Darmstadt) werden in 50 ml destilliertem Wasser gelöst, mit 0,2 g Bromcyan bei pH 11,0 30 Minuten aktiviert und danach mit 2 n HCl auf pH 7,0 eingestellt. Es werden 20 ml Saccharose-Mutase-Lösung mit 10 000 Einheiten 2 Stunden bei Raumtemperatur und pH 6,5-7,0 gekuppelt. Der lösliche Enzymträger wird über eine Amicon XM 100-Membran von Saccharose-Mutase getrennt und mehrmals mit insgesamt 500 ml 0,1M Phosphatpuffer pH 7,0 gewaschen.
150 ml Stärke-Saccharose-Mutase mit 7 020 Einheiten ≙ 70,2 % der eingesetzten Aktivität.

Beispiel 14

Kovalente Bindung von Saccharose-Mutase an Dextran 60 nach Aktivierung mit Bromcyan

2,5 g Dextran 60 (Molekulargewicht 60 000-90 000, Fa. Serva, Heidelberg) werden wie in Beispiel 13 angegeben mit Bromcyan aktiviert und mit 102 ml Saccharose-Mutase-Lösung (12 500 Einheiten und 250 mg Protein nach Lowry) gekuppelt und wie unter 3.1. angegeben aufgearbeitet.
104 ml Dextran 60-Saccharose-Mutase mit 8 580 Einheiten ≙ 68,6 % der eingesetzten Aktivität.

Beispiel 15

Kovalente Bindung von Saccharose-Mutase an Manucol LD[R] nach Aktivierung mit Bromcyan

2,5 Manucol LD[R] (lösliches Alginat der Fa. Alginate Industries GmbH, Hamburg) werden wie in Beispiel 13 angegeben mit Saccharose-Mutase gekuppelt und aufgearbeitet.
192 ml Manucol-Saccharose-Mutase mit 11 712 Einheiten ≙ 93,7 % der eingesetzten Aktivität.
Manucol-Saccharose-Mutase kann nach Eintropfen in 1 %ige CaCl₂-Lösung als unlöslicher Enzymträger ausgefällt werden.

Beispiel 16

Kovalente Bindung von Saccharose-Mutase an Carrageenan nach Aktivierung mit Bromcyan

2,5 g Carrageenan (löslicher Polysaccharidschwefelsäureester aus der Seealge Chandrus crispus, Fa. Serva, Heidelberg) werden wie in Beispiel 13 angegeben mit Saccharose-Mutase gekuppelt und aufgearbeitet.
210 g Carrageenan-Saccharose-Mutase mit 8 421 Einheiten ≙ 67,4 % der eingesetzten Aktivität.

Beispiel 17

Kovalente Bindung von Saccharose-Mutase an ein lösliches Anhydrid-Polymer

0,5 g Gantrez AN 179[R] (lösliches Copolymer aus Maleinsäureanhydrid und Methylvinylether, Fa. Serva, Heidelberg) werden in 50 ml destilliertem Wasser gelöst, 12 250 Einheiten Saccharose-Mutase zugesetzt und die Lösung bei pH 6,2 bei Raumtemperatur über Nacht gerührt. Nicht gebundenes Enzym wird durch Ultrafiltration über eine Amicon XM 100-Membran abgetrennt.

310 ml Gantrez AN 179-Saccharose-Mutase mit 3 040 Einheiten $\hat{=}$ 24,3 % der eingesetzten Aktivität.

Beispiel 18

Adsorptive Bindung von Saccharose-Mutase an Titandioxid mit anschließender Quervernetzung mittels Glutardialdehyd

10 g Enzacryl-Titandioxid (Koch-Light Laboratories Ltd., Colnbrook, England) werden in 10 ml destilliertem Wasser suspendiert und mit 4 ml Saccharose-Mutase-Lösung bei pH 6,5 gerührt. Der Enzymträger wird abgesaugt und mit destilliertem Wasser gewaschen.

Ausbeute an Enzymträger : 12,88 g Titandioxid-Saccharose-Mutase mit 22,3 Einheiten/g = 286,3 Einheiten.

Filtrat : 75 ml mit 448,5 Einheiten

Der Titandioxid-Saccharose-Mutase-Träger kann durch 30-minütige Behandlung mit 0,1 %iger Glutardialdehyd-Lösung vernetzt werden.

Beispiel 19

Umwandlung von Saccharose in Isomaltulose mit adsorbierter Saccharose-Mutase

a) 21,3 g des trägergebundenen Enzyms aus Beispiel 3 werden in einer Säule 1,6 × 14 cm bei 30 °C mit einer 10 %igen Saccharose-Lösung durchströmt. Bei einem Durchfluß von 60 ml/Std. wird durch HPLC-Analyse folgende Zusammensetzung des Säulenablaufes gefunden :
80,7 % Isomaltulose, 0 % Saccharose, 7,4 % 1-O-α-D-glucopyranosido-D-fructose, 6,9 % Fructose, 5 % Glucose.

b) In der gleichen Säule wird 60 %ige Saccharose-Lösung mit einem Durchfluß von 20 ml/Std. umgewandelt. HPLC-Analyse des Ablaufes :
85,6 % Isomaltulose, 0 % Saccharose, 10,6 % 1-O-α-D-glucopyranosido-D-fructose, 3,1 % Fructose, 0,7 % Glucose. Der Säulenreaktor wurde über eine Zeit von 540 Stunden mit einem Aktivitätsverlust von weniger als 30 % betrieben.

c) In gleicher Weise wurde die Reaktion mit 60 %iger Saccharose-Lösung bei 45 % und einem Durchfluß von 30 ml/Std. durchgeführt. Unter diesen Bedingungen ging der Umsatz in 430 Stunden auf 50 % zurück.

Beispiel 20

Umwandlung von Saccharose in Isomaltulose mit kovalent gebundener Saccharose-Mutase

50 ml eines feuchten Präparates trägergebundener Saccharose-Mutase nach Beispiel 8 werden in eine Säule 2 × 17 cm gefüllt und von einer 48 % Saccharose-Lösung mit 11 ml/Std. durchströmt. Die Reaktion wird zuerst 500 Stunden bei 35 °C, dann weitere 330 Stunden bei 45 °C durchgeführt. Der Säulenablauf hat typischerweise die Zusammensetzung :
80 % Isomaltulose, 6 % Saccharose, 8 % 1-O-α-D-glucopyranosido-D-fructose, 4 % Fructose, 2 % Glucose. Über die gesamte Versuchsdauer ist der Aktivitätsverlust des Reaktors weniger als 20 %.

**Ansprüche**

1. Aus Bakterien isoliertes Enzym, das Saccharose als Hauptprodukt in Isomaltulose und daneben als wichtigste Nebenprodukte in 1-O-α-D-Glucopyranosido-D-fructose, Fructose und Glucose, jedoch in keine nachweisbaren Mengen anderer Disaccharide umwandelt.

2. Enzym nach Anspruch 1, welches aus Protaminobacter gewonnen wird.

3. Immobilisiertes Enzym, das durch Immobilisierung eines Enzyms nach Anspruch 1 oder 2 gewonnen wird.

4. Immobilisiertes Enzym nach Anspruch 3, dadurch gekennzeichnet, daß die Immobilisierung durch Einschluß in Hohlfasern erfolgt.

5. Immobilisiertes Enzym nach Anspruch 3, dadurch gekennzeichnet, daß die Immobilisierung durch Adsorption an einen festen Träger erfolgt.

6. Immobilisiertes Enzym nach Anspruch 3, dadurch gekennzeichnet, daß die Immobilisierung durch kovalente Bindung an einen festen Träger erfolgt.

7. Immobilisiertes Enzym nach Anspruch 3, dadurch gekennzeichnet, daß die Immobilisierung durch kovalente Bindung an einen löslichen Träger erfolgt.

8. Immobilisiertes Enzym nach Anspruch 3, dadurch gekennzeichnet, daß die Immobilisierung durch Bindung an eine Membran erfolgt.

9. Verfahren zur Herstellung von Isomaltulose aus Saccharose, dadurch gekennzeichnet, daß man ein immobilisiertes Enzym nach Anspruch 3-8 verwendet und die Reaktion bei 30-60 °C und einer Saccharose-Konzentration von 10-75 %, vorzugsweise 40-60 %, chargenweise oder kontinuierlich durchführt.

10. Verfahren nach Anspruch 9, dadurch gekennzeichnet, daß man ein immobilisiertes aus Protaminobacter gewonnenes Enzym verwendet.

## Claims

1. An enzyme which has been isolated from bacteria and converts sucrose into isomaltulose as the main product and in addition into 1-O-α-D-glucopyranosido-D-fructose, fructose and glucose as the most important by-products, but into no detectable quantities of other disaccharides.

2. The enzyme according to Claim 1, which is isolated from Protaminobacter.

3. Immobilised enzyme which is isolated by immobilising an enzyme according to Claim 1 or 2.

4. Immobilised enzyme according to Claim 3, characterised in that the immobilisation is carried out by inclusion in hollow fibres.

5. Immobilised enzyme according to Claim 3, characterised in that the immobilisation is carried out by adsorption onto a solid carrier.

6. Immobilised enzyme according to Claim 3, characterised in that the immobilisation is carried out by covalent bonding to a solid carrier.

7. Immobilised enzyme according to Claim 3, characterised in that the immobilisation is carried out by covalent bonding to a soluble carrier.

8. Immobilised enzyme according to Claim 3, characterised in that the immobilisation is carried out by bonding to a membrane.

9. Process for the preparation of isomaltulose from sucrose, characterised in that an immobilised enzyme according to Claims 3-8 is used, and the reaction is carried out batchwise or continuously at 30-60 °C and at a sucrose concentration of 10-75 %, preferably 40-60 %.

10. Process according to Claim 9, characterised in that an immobilised enzyme isolated from Protaminobacter is used.

## Revendications

1. Enzyme isolée à partir de bactéries, qui convertit le saccharose en isomaltulose comme produit principal et, à côté, en tant que sous-produits les plus importants, en 1-O-α-D-glucopyranosido-D-fructose, fructose et glucose, mais non en d'autres disaccharides en quantités décelables.

2. Enzyme selon la revendication 1, obtenue à partir de Protaminobacter.

3. Enzyme immobilisée, obtenue par immobilisation d'une enzyme selon la revendication 1 ou 2.

4. Enzyme immobilisée selon la revendication 3, caractérisée en ce que l'immobilisation se fait par inclusion dans des fibres creuses.

5. Enzyme immobilisée selon la revendication 3, caractérisée en ce que l'immobilisation se fait par adsorption sur un support solide.

6. Enzyme immobilisée selon la revendication 3, caractérisée en ce que l'immobilisation se fait par liaison covalente sur un support solide.

7. Enzyme immobilisée selon la revendication 3, caractérisée en ce que l'immobilisation se fait par liaison covalente sur un support solide.

8. Enzyme immobilisée selon la revendication 3, caractérisée en ce que l'immobilisation se fait par liaison sur une membrane.

9. Procédé de fabrication d'isomaltulose à partir de saccharose, caractérisé en ce qu'on utilise une enzyme immobilisée selon les revendications 3 à 8 et en ce qu'on exécute la réaction à 30-60 °C à une concentration de saccharose de 10-75 %, de préférence de 40-60 %, par charges ou en continu.

10. Procédé selon la revendication 9, caractérisé en ce qu'on utilise une enzyme immobilisée récupérée à partir d'un Protaminobacter.

FIG. 1